# EUROPEAN PATENT SPECIFICATION

(11) **EP 3 173 067 B1**
(45) Date of publication and mention of the grant of the patent: **14.11.2018**
(21) Application number: 15194799.1
(22) Date of filing: 16.11.2015
(51) Int. Cl.: A61K 9/00, A61K 47/36, A61K 31/167, A61K 31/573, A61K 31/7048, A61K 31/732

(54) **MUCOADHESIVE BUCCAL IN SITU GEL FORMULATION**
MUCOADHÄSIVE BUKKAL IN SITU GELFORMULIERUNG
GINGIVAL MUCO-ADHESIF IN SITU GEL FORMULATION

(43) Date of publication of application: 31.05.2017
(73) Proprietor: Istanbul Universitesi Rektorlugu, Istanbul (TR)
(72) Inventor: Yildiz Peköz, M Ayca, 34330 Istanbul (TR); Özsoy Erginer, M Yildiz, 34116 Istanbul (TR); Arslan, Derya, Istanbul (TR)
(74) Representative: Mutlu, Aydin

(56) References cited:
- EP-A1- 1 236 466
- WO-A1-03/094894
- WO-A2-2004/041118

## Description

### Technical Field of the Invention

The present invention is related to *in situ* gelling formulations suitable to use in oral diseases, especially in the treatment of oral candidiasis or aphtha. The current invention is more particularly related to *in situ* gelling formulations to be applied via buccal route which comprises nystatin, corticosteroid and at least one local anaesthetic agent. The gel formulations according to this invention cover the mouth cavity by being gelled at body temperature by means of using polymers being liquid at room temperature and gelling at body temperature when it is sprayed into the mouth, and which can be adhered into the oral cavity by means of mucoadhesive polymers. The gelling formulations of the current invention provide improved release of the active agent and effective treatment in the above mentioned diseases.

### Background of the Invention

Candidiasis, is the leading common opportunistic infections in patients infected with HIV/AIDS, due to the suppression of immune system and use of antitumoral drugs in cancer patients *[*Feigal, D.W., Katz, M.H., Greenspan, D., Westenhouse, J., Winkelstein, W., Lang, W., Samuel, M., Buchbinder, S.P., Hessol, N.A., Lifson, A.R. (1991). The prevalence of oral lesions in HIV-infected homosexual and bisexual men: three San Francisco epidemiological cohorts. AIDS, 5, 519-525*.].* The majority of these infections are caused by *Candida albicans.* These infections can occur locally as well as systemically. Oral candidiasis is a local and widely seen candida infection. Aphtha, another disease occurred in the mouth, is also named as "aphthous ulcer" and is an ulcer formation with pain occurring in the mouth. A typical aphtha sore is observed as red oval restricted sores on the mucosa layer in the mouth.

Antifungal drugs are used in the treatment of these infections. Amphotericin B and fluconazole are the most preferred antifungals orally applied in conventional treatment *[*Burgess, D.S., Hastingsa, R.W., Summersa, K.K., Hardina, T.C., Rinaldid, M.G. (2000). Pharmacodynamics of fluconazole, itraconazole, and amphotericin B against Candida albicans. Diagnostic Microbiology and Infectious Disease, 36, 13-18*].* However, in some studies, the development of resistance to these drugs has been identified. Additionally, the administration of these drugs via systematic route exposes patients to toxicity. Also, some studies in recent years indicate that the local antifungal treatment provides more efficient recovery than systematic treatment *[*Sudhakar, Y., Kuotsu, K., Bandyopadhyay, A.K. (2006). Buccal bioadhesive drug delivery - A promising option for orally less efficient drugs. J Contr Rel, 114, 15-40*.].* Therefore, nystatin, a polyene macrolide antifungal which can be applied only locally, is found to be preferable in the context of the present invention. Nystatin, by binding to cell membrane, is effective on the permeability and transport functions; and the daily dose thereof is 2-4 mg/kg in topical use. Nystatin is used in fungal infections in the mouth; it has no absorption in the stomach. Hence, pastille or oral suspension forms of nystatin are available. However, the short endurance of these formulations in the mouth poses a disadvantage in therapy. In order to solve this problem, bioadhesive dosage forms are developed so that the active agent can be kept longer in the buccal area.

In recent years, many adhesive mucosal dosage forms including primarily adhesive tablets, gels, patches and polymeric films have been developed *[*Perioli, L., Ambrogi, V., Angelici, F. et. al. (2004). Development of mucoadhesive patches for buccal administration of ibuprofen. J Contr Rel, 99, 73-82*.].* The expected features from buccal drug release systems are to have flexible and good bioadhesive properties and as such to ensure keeping of the drug in the oral cavity for a desired period of time. Moreover, the therapeutic response required is provided by means of the release of the drug in a controlled and predictable manner by buccal systems in mucoadhesive structure *[*Wong, C.F., Yuen, K.H., Peh, K.K. (1999). Formulation and evaluation of controlled release eudragit buccal patches. Int.J.Pharm, 178, 11-22*.].* In recent years, controlled drug release technologies are increasingly gaining importance. The reduction in dose frequency, accordingly, enhancement in patient compliance, minimization of side effects, acquisition of longer-term effects and reduction of treatment costs are among the advantages of these systems *[*Wise, D.L. (Ed.). (2000). Handbook of Pharmaceutical Controlled Release Technology. New York,: Marcel Dekker Inc., 465-466*.]*.

The short retention time of nystatin, having a strong antifungal activity against Candida albicans, at the application site has led to the requirements of designing new formulations relating to this active agent. The concentration of nystatin in oral secretion should be higher than the minimum inhibitory concentration (MIC) thereof in order that the treatment with nystatin be successful. In recent years, it is thought that this problem can be overcome by understanding the effectiveness of controlled release systems and use of polymers in mucoadhesive structure. In this respect, various mucoadhesive buccal formulations of nystatin prepared with bioadhesive polymers are available in the literature. However, these studies do not offer required physical form or formulations providing effective treatment for mouth diseases like aphtha. Furthermore, the limited anti-inflammatory and anaesthetic properties of formulations in the prior art presents another disadvantage.

In this connection, a difficulty encountered in this type of formulations is the further increase of pain and irritation during the disease process proceeding with already severe symptoms caused by the drug-drug interactions and cross reactions in the presence of active agents other than nystatin. Said pain and irritation should not be confused with the pain and irritation resulted from the disease itself. Because, the additional pain and irritation caused by drug-drug interaction and adverse reactions reveal themselves in time. Therefore, another objective of the invention is to provide *in situ* gelling buccal formulations comprising at least one corticosteroid and a local anaesthetic agent in addition to nystatin.

Another problem detected in such oral film formulations comprising multiple active agents is the difficulties encountered during adjusting release profiles of active agents in one formulation due to the fact that different release profiles are desired for each of the active agents used. Hence, another objective of the invention is to provide oral film formulations having suitable multiple active agent combinations which display the desired release profile.

The formulations of the current invention are provided in the form of *in situ* gelling buccal formulation, with the aim of bringing a solution to the above mentioned problems, comprising nystatin having antifungal effect against the infections occurred in the mouth, together with a corticosteroid to eliminate the inflammation, and at least one local anaesthetic in order to relieve the pain and irritation. Said formulations *in situ* form meets a need in the prior art due to their mucoadhesive gel characteristic having a wide spread area when it is applied into the mouth, being liquid at room temperature. Thereby, it is intended to provide antifungal treatment and the treatment of other symptoms caused by fungi infection at the same time. Therefore, the pharmaceutical formulation of the invention comprises different active agents in addition to nystatin contrary to other studies comprising single active agent. Also, as a new approach in the present invention, new formulations are developed by using polymers gelling at body temperature and mucoadhesive polymers. These polymers are considered to be effective in obtaining formulations which are gelled at body temperature and can be adhesive to mouth mucosa, can remain there for a long time in the presence of three different active agents. Gel formulations can adhere to the mucosa and release the active agents for a long time *[*Shin SC, Kim JY (2000). Enhanced permeation of triamcinolone acetonide through the buccal mucosa. Eur J Biopharm, 50, 217-220*].*

### Brief Description of the Invention

The present invention is related to a pharmaceutical composition in buccal form comprising nystatin, at least one corticosteroid and at least one anaesthetic agent with at least one *in situ* gelling polymer and at least one mucoadhesive polymer.

At least one corticosteroid in the pharmaceutical composition can be selected from a group comprising hydrocortisone, cortisone, tixocortol, prednisolone, prednisone, methylprednisolone, triamcinolone, mometasone, amcinonide, budesonide, desonide, fluocinonide, fluosinilo, halcinonide, betamethasone, dexamethasone, fluocortolone.

In a preferred embodiment of the invention, at least one corticosteroid in the composition is hydrocortisone, more preferably hydrocortisone acetate.

A further preferred feature of the pharmaceutical compositions according to the invention is that at least one local anaesthetic is selected from a group comprising mepivacaine, etidocaine, lidocaine, prilocaine, bupivacaine, procaine.

In a preferred embodiment of the invention, at least one local anaesthetic agent used is lidocaine, preferably HCl salt and/or free base thereof.

In another embodiment of the invention, *in situ* gelling polymer used in the formulations according to the invention is selected from a group comprising gellan gum, alginic acid, xyloglucan, pectin, chitosan, poly(DL-lactic acid), poly(DL-lactide-co-glycolide), poly-caprolactone, poly(N-isopropyl acrylamide) [poly (NIPAAM)], polyoxyethylene, polyoxypropylene and combinations thereof.

In a preferred embodiment of the invention, *in situ* gelling polymer used in buccal formulations prepared according to the present invention is a poloxomer, such as a mixture of polyoxyethylene and polyoxypropylene, and more preferably of poloxomer 407 and poloxomer 188, or a mixture thereof. Both poloxomers are commercially available, for instance from BASF under the tradename Kolliphor® P407 and Kolliphor® P188, respectively.

Mucoadhesive polymers used in the formulations of the invention can be selected from a group comprising cellulose derivatives such as methyl cellulose, ethyl cellulose, hydroxyethyl cellulose, hydroxypropyl cellulose, hydroxypropyl methylcellulose, sodium carboxymethylcellulose, polyacrylic acid polymers such as carbomers and polycarbophils, poly (hydroxyethyl methacrylate), poly (ethylene oxide), poly (vinyl pyrrolidone), poly (vinyl alcohol), sodium alginate, karaya gum, guar gum, xanthan gum, gelatine, pectin and chitosan and combinations thereof.

In a preferred embodiment of the invention, mucoadhesive polymer used in the formulations is Carbopol 934 being a polyacrylic acid polymer, commercially available from Merck.

In another embodiment of the invention, the polymers forming *in situ* gelling polymer mixture used in the formulations of the current invention comprise poloxomer 407 and poloxomer 188 with a ratio between 25:2 and 10:2, preferably from 22:2 to 16:2 by weight.

In another embodiment of the invention, the mucoadhesive polymer ratio in the composition is between 0.1 to 5 %, preferably 0.1 to 1 % by weight.

In a further embodiment of the invention, the total polymer ratio used in the composition is 10 to 25 %, preferably 12 to 20 % by weight.

In another aspect, the buccal formulations provided according to the invention comprise at least one surface active agent in addition to *in situ* gelling polymers and mucoadhesive polymers. Said surface active agents are selected from a group comprising Tween 80, ascorbic palmitate and Cremophor RH 40 and combinations thereof.

In a further aspect, the current invention is related to a pharmaceutical formulation for use in the treatment of oral candidiasis or aphtha.

### Detailed Description of the Invention

The present invention is related to *in situ* gelling formulations being applied via buccal route, for use especially in fungal infections like aphtha, comprising nystatin having antifungal effect together with at least one corticosteroid in order to eliminate the inflammation formed, and at least one local anaesthetic in order to eliminate the pain.

Because of the agents of 3 different pharmacological groups used in the formulations of the invention, it is aimed to treat other symptoms caused by fungal infection together with antifungal treatment. In the formulations according to the invention, it is determined that there exists some advantageous effects such as the provision of the contact of active agents for a long time with the mucosa by means of the system comprising 3 different pharmacological groups which is liquid at room temperature but can be sprayed into the mouth, reaches to a wide application zone and is gelling at that zone.

The current invention aims to prolong the retention time in the mouth of the formulations in prior art such as pastille or oral suspension through which they show their effect and thus enhance the effect of active agent(s). For this reason, *in situ* gelling polymers sensitive to body temperature are used in the preparation of buccal formulations of the invention.

*In situ* gels are drug carrier systems which are liquid prior to application and are converted into gel form after application. *In situ* gels can be applied via oral, ocular, vaginal, injectable and intraperitoneal routes. The most important advantages of *in situ* gels is that they provide easiness of application, enhance the bioavailability, decrease the dose concentration and frequency and improve the patient compliance and comfort. Also, *in situ* systems are not too complex and therefore the product development is easy and the production costs are low *[*Tejas, G., Vishal, G., Vishal, C., Umesh, UA (2011). Review on novel in situ polymeric drug delivery system. Int J Pharm Res and Dev. 3, 53-59*]. In situ* gel formation is realized through various mechanisms such as solvent modification, UV radiation, ionic cross linking, pH modification and adjustment of temperature *[*Ruel-Gariépy, E., Leroux, J-C. (2004). In situ forming hydrogels-review of temperature sensitive systems Eur J Pharm and Biopharm, 58,409-426*].* It is preferred to use polymers sensitive to temperature and ions for *in situ* gel formulations being applied via oral route. Poloxomers are block polymers composed of polyoxyethylene-polyoxypropylene-polyoxyethylene type block polymers and comprises 70% nonionic surface active agent polyoxyethylene unit *[*Cho, CW., Shin, SC., Oh, IJ (1997) Thermorheological properties of aqueous solutions and gels of poloxomer 407. Drug Dev Ind Pharm a 23, 1227-1232*].* In the exemplary studies of the present invention, poloxomer 407 having polyethyleneoxide (PEO): polypropyleneoxide (PPO) ratio of 7:3 and average molecular weight 12600 Da was chosen. The aqueous solutions of Poloxomer 407 display reversible thermal gelling at a concentration of 20-30% (w/w) *[*Schmolka, IR. (1972) Artifical skin I. Preparation and properties of Pluronic F127 gels for treatment of burns. J Biomed Mater Res 6, 571-582*]*. Moreover, the analogue of Poloxomer 407 which is Poloxomer 188 is added to the formulation during temperature adjustment. Poloxomer 188 increases the gelling temperature of Poloxomer 407.

At least one *in situ* gelling polymer used in the buccal formulations of the invention is preferably selected from a group comprising gellan gum, alginic acid, xyloglucan, pectin, chitosan, poly (DL-lactic acid), poly(DL-lactide-co-glycolide), poly-caprolactone, poly(N-isopropyl acrylamide) [poly (NIPAAM)], polyoxyethylene, polyoxypropylene and combinations thereof. More preferably, at least one *in situ* gelling polymer used in the formulations of the invention can be selected among poloxomers, said *in situ* gelling polymers are more preferably poloxomer 188 and 407.

Another desired characteristic of the formulations according to the current invention is the adherence of *in situ* gel to the mucosa. Mucoadhesion increases the drug release specific to a region due to the incorporation of the pharmaceutical formulations comprising active agent with mucoadhesive hydrophilic polymers. The reason for this formulation to be adhered on a biological surface is the localized drug release. A release of the active compound close to the action zone increases significantly the pharmaceutical effect *[*Woodley, J. (2001). Bioadhesion, new possibilities for drug administration? Clin Pharmacokinet, 40 (2), 77-84*.]*.

Mucoadhesive polymers used in the buccal formulations prepared according to the present invention are selected from a group comprising cellulose derivatives such as methyl cellulose, ethyl cellulose, hydroxyethyl cellulose, hydroxypropyl cellulose, hydroxypropyl methylcellulose, sodium carboxymethylcellulose, polyacrylic acid polymers such as carbomers and polycarbophils, poly (hydroxyethyl methacrylate), poly (ethylene oxide), poly (vinyl pyrrolidone), poly (vinyl alcohol), sodium alginate, karaya gum, guar gum, xanthan gum, gelatine, pectin and chitosan and combinations thereof. At least one mucoadhesive polymer used in the buccal formulations of the invention is preferably Carbopol 934. Carbopol 934 is a very hydrophilic polyacrylic acid polymer and is preferred because it displays high viscosity and good bioadhesive property at low concentration in the preparation of gel formulation for topical application *[*Robinson JR., Moyne GM (1995) Bioadhesive and phase-change polymers for ocular drug delivery. Adv. Drug Deli Rev 16, 45-50*].* Also, Carbopol being dependent on pH, is in solution form at acidic pH and is in gel form with low viscosity at alkali pH *[*Ismail, FA., Rapaport J, Hughes JA, Brazean GA. (2000) In situ gel Formulation for gene delivery: release and myotoxicity studies. Pharm Dev Technol 5, 391-397*]*.

As it is already stated above, one of the important characteristics of the formulation of the invention is to contain in itself three different active agents, namely nystatin, at least one corticosteroid and at least one local anaesthetic and thus to provide three different treatments at the same time in single dosage form.

Among the corticosteroids mentioned in the scope of the invention, having well-known anti-inflammatory characteristics in the art, hydrocortisone acetate is preferably used according to the invention. Again, the local anaesthetic mentioned above can be any local anaesthetic having transdermal effectiveness in prior art. In the scope of the invention, lidocaine free base or HCl salt thereof can be used. Lidocaine HCl is highly soluble in water, but its free base form is not soluble in water due to its hydrophobicity. The inventors have found thereby that the gelling properties in the oral cavity can be adjusted as desired by using the mixtures of lidocaine free base and salt forms thereof.

Nystatin, being one of the active agents, is fungistatic and also fungicidal. Clinically, in the topical use, it has wide spectrum in the treatment of mucocutaneous *Candida* infections such as C. *albicans, C. parapsilosis, C. krusei* and C. *tropicalis [*Kurifilm, T., Williams, D.W., Bagg, J. (2005). In vitro susceptibility of oral Candida to seven antifungal agents. Oral Microbiol Immunol, 20(6), 349-53*.]*. The absorption of nystatin from the skin, mucosa membrane and gastrointestinal organs are quite low and negligible *[*Bennett, J.E. (1993). Antimicrobial agents: antifungal agents. Gilaman, A.G., Nies, A.S. (Ed), The pharmacological basis of therapeutics. New York: McGraw-Hill; 1165-81*.]*. Nystatin is a yellow or slightly brownish hygroscopic powder. Practically, it is not soluble in water and alcohol, and is freely soluble in dimethyl formamide and dimethyl sulfoxide. It is slightly soluble in methanol [Ph. Eur. 7.0]. The pH of its 3% solution is 6.0 - 8.0 [USP 37]. The logP value is -2.7, and pKa value is 12.65.

Lidocaine is one of the most preferred agents in surface anesthesia. It is a white or closest to white crystal powder. It is highly soluble in water and freely soluble in ethanol. The pH of its 5 mg/ml solution in water without carbon dioxide is 4.0 - 5.5 [Ph. Eur. 7.0]. Local anaesthetics block sensory and motor functions by reversibly binding to voltage gated and sodium gated ion channels *[*Heavner, J.E. (2007). Local anaesthetics. Curr Opin Anaesthesiol, 20, 336-342*.]*.

Hydrocortisone acetate is white or closest to white powder form. Its taste is bitter and it is odourless *[*Florey, K. Hydrocortisone. K. Florey (Ed.), Analytical Profiles of Drug Substance. Vol. 12., American Pharmaceutical Association, 277-324*.].* Practically it is insoluble in water. It is slightly soluble in ethanol without water and methylene chloride [Ph. Eur. 7.5]. It is the most important natural glucocorticoid secreted from adrenal cortex. It is used in the treatment of allergic conjunctivitis, alopecia, anterior segment inflammation, atopic dermatitis, insect bites and stings, bursitis, inflammation of the skin, Crohn's disease, dermatitis herpetiformis, discoid lupus erythematosus, exfoliative dermatitis, endophthalmitis, epicondylitis, erythema multiforme, granuloma annulare, Graves ophthalmopathy, gouty arthritis, haemorrhoids, uveitis, juvenile rheumatoid arthritis, itching (pruritus), keratitis, contact dermatitis and many other diseases.

Preferably, Poloxomer 188 and 407 are used as a polymer gelling agent at body temperature and Carbopol 934 is used as mucoadhesive polymer in the content of the *in situ* gelling buccal formulations provided in the scope of the present invention. Said formulations can comprise inactive ingredients in addition to three active agents, *in situ* gelling polymer and mucoadhesive polymer. Said inactive ingredients can be selected from a group comprising solvent and surface active agents. The solvents used in the formulations according to the present invention can be selected from a group comprising water, ethyl alcohol and combinations thereof. The surface active agent used in the formulations of the invention can be selected from a group comprising Tween 80, Cremophor RH40 and ascorbic palmitate.

Since the release of the active agent is realized through matrix and erosion mechanisms from the polymers used in the *in situ* gelling buccal formulations according to the invention, it is aimed that lidocaine and hydrocortisone show their effects in the first hours and their effect is continued for a long time. It is very important that pain and inflammation symptoms are treated in a fast way and that the treatment lasts for a long time for oral candidiasis for the sake of patient compliance. The fact that nystatin displays controlled release and the release amount is above the MIC value because of the gelling properties of the polymers used, physicochemical characteristic of nystatin used as antifungal agent, and the release that increasingly continues are some strong characteristics of this invention. *In situ* gelling buccal formulation in addition to desired release profile should also provide some characteristics such as gelling at body temperature when it is sprayed into the mouth which is liquid at room temperature and adherence to mucosa. The fact that all these elements are provided at the same time in a system where there are three different active agents presents some difficulties.

The inventors have showed that in case a combination comprising nystatin, hydrocortisone acetate and lidocaine HCl is arranged as *in situ* gelling buccal formulation, it can be applied in the treatment of the diseases observed in the mouth, especially such as oral candidiasis and aphtha and that it can be effective for a long time by adhering to the mucosa. However, as it can be appreciated by the experts in the technical field, that kind of *in situ* formulation can find different application areas. For instance, the use of mucoadhesive *in situ* gels according to the invention is possible in the treatment of diseases and defects related to teeth.

Accordingly, the inventors, in their studies performed, have found that in the case of a combination with three different active agents, at least one *in situ* gelling polymer and at least one mucoadhesive polymer are used in mucoadhesive film forms gelling at body temperature and adhering to mouth mucosa can be developed. It is known that each chemical agent added to the content of the formulation can modify the gelling temperature of *in situ* gel formulation, for this reason, as a new approach in the formulations of the invention, the effect of the 3 active agents and the polymer types and ratios used together with said 3 active agents in the formulation are evaluated. As a result of the studies performed, it is realized that the effect of the ratio of the polymers used in the formulations of the invention to each other and to that of the active agents may substantially affect the desired gelling temperature and the residence time in the mouth.

According to this, in another aspect, *in situ* gelling polymers in the buccal formulations of the present invention, the Poloxomer 407: Poloxomer 188 ratio is from 25:2 to 10:2, preferably from 22:2 to 16:2, by weight. The *in situ* gelling polymers in this specific aspect, can be arranged such as Poloxomer 407: Poloxomer 188 is 9:1; 9.5:0.5; 9.7:0.3; 9.9:0.1; or 9.15:0.85, by weight. On the other hand, the ratio of mucoadhesive polymer used in said buccal formulations is between 0.1 to 1 %, preferably 0.1 to 0.5 % on the basis of the total weight of the composition. The mucoadhesive polymer used in the buccal formulations according to the invention is preferably Carbopol 934 commercially available from Merck.

In another aspect, the ratio of the polymer and/or polymer mixtures used in the buccal formulations of the present invention is 10 to 30%, preferably 12 to 25%, more preferably 15 to 18% by weight on the basis of the total weight of the composition.

The *in situ* gelling buccal formulations may further comprise at least one surface active agent. The inventors have surprisingly found that the release profile of nystatin which is insoluble in water can be improved by the use of a surface active agent. It is determined that the solubility of the main active agent nystatin, despite its insolubility in water, is increased by means of using surface active agent. The release is realized in matrix structured polymer systems by the diffusion of the water inside followed by its diffusion to outside. Even though the water is diffused into the matrix structure, the fact that the active agent is not sufficiently soluble in water causes not to obtain the sufficient efficacy. The surface active agent added to the formulation increases the solubility of the active agent. To this end, as the surface active agent; Tween 80, ascorbic palmitate and Cremophor RH 40 or combinations thereof can be preferred. In the scope of the formulations according to the invention, Cremophor RH 40 and Tween 80 are particularly preferred.

In another aspect, the invention is related to a pharmaceutical formulation according to the present invention for use in the treatment of oral candidiasis and aphtha.

At this point, the formulations of the present invention can comprise lidocaine free base, HCl salt thereof or a mixture of these two. The preformulation studies presented below, even though they are exemplified with hydrocortisone and lidocaine according to the preferred embodiments, should not be interpreted as limited to the scope of the invention.

### Examples

### Preformulation Studies:

### Step 1. Determination of the gelling temperatures of gels by using a single poloxomer

### Preparation Method I:

To prepare the systems gelling with temperature, Poloxomer 407 (Kolliphor® P407, BASF) polymer dispersions are prepared at 6 different concentrations (15%, 16%, 18%, 20%, 22%, 25% (w/w)) by being swelled in water. Poloxomers swell faster in cold water therefore the systems are prepared by swelling in cold method. At first stage, poloxomers 407 are added as portions into distilled water (0-4°C) in a beaker placed in ice bath, they are swelled slightly by being kept in ice bath, and were mixed for 2-4 hours with the help of a magnetic stirrer (homogenous-clear appearance). Then, it was left in the refrigerator for being swelled for 24 hours. The prepared formulations are evaluated based on their gelling temperatures, whereby the temperature at which the system loses its flow properties is measured when the system is under magnetic stirring. During the preparation, because the formulations comprising Poloxomer 407 at ratios 20%, 22% and 25% (w/w) lose their flow properties, it is assumed that their gelling temperatures are under 25°C. For this reason, it is decided to continue with the studies with polymer mixtures having poloxomer 407 ratios 15%, 16% and 18% (w/w) and gelling temperatures 37.57±1.60, 33.17±0.40 and 28.4±0.76 respectively.

### Step 2. Determination of gelling temperatures of gels by using two poloxomers

It is noted that Poloxomer 188 (Kolliphor® P188, BASF) increases the gelling temperature. The gelling temperatures of each of the three formulations are measured by adding each of the three formulations with different amounts of 15 % (w/w) Poloxomer 188 solution.

At first stage, the formulations having been observed with gelling temperatures in the range of 31-45°C were selected. It is decided to observe the change in the gelling temperatures of these formulation after the addition of polymer which increases the adhesion.

### Step 3. Addition of Carbopol

Carbopol 934® (Merck) is added to selected formulations at ratios 0.05%, 0.1%, 0.2% and 0.3% (w/w) 2%, 4% and 6% (w/w). The obtained data shows that the high concentrations of Carbopol 934® affect the gelling temperature significantly or create problem in the preparation method. However, gelling temperature is found in the range of 33.3-31.6°C by the addition of 0.2% (w/w) Carbopol 934®. In the light of these data, it is decided to study the formulations comprising Carbopol 934® with an optimum amount of 0.2% (w/w) or below.

### Step 4. Addition of Active Agents

Mixture of the active agents is added to the formulations whose gelling temperatures are selected at the end of step 2 and with the addition of Carbopol 934 (0.2% (w/w)) at the end of step 3. A decrease in the gelling temperatures of some formulations is observed during the addition of active agents. Final formulations are selected among the formulations having gelling temperatures between 34±2°C.

Moreover, it is decided to add a surface active agent to the formulations. Cremophor RH 40 and Tween 80 (TW) were selected and the concentrations of these surface active agents in the formulations are determined. Final formulations are prepared with decided polymer and surface active agent percentages. The considered parameters and results obtained in these studies are presented below.

There was no change in the gelling temperatures even after *in situ* gelling buccal formulations are kept at room temperature for 7 days.

*In situ* formulations comprising active agents at the optimized conditions based on the studies above are presented in the Table 1 below.

### Preparation Method II (Hot Method)

### Step 1. Determination of gelling temperatures of gels by using two poloxomers

To prepare the systems gelling with temperature, Poloxomer 407 polymer dispersions are prepared at 6 different concentrations (15%, 16%, 18%, 20%, 22%, 25% (w/w)) and 15 % (w/w) Poloxomer 188 solution by being swelled in water. Poloxomers swell faster in hot water (70°C) therefore the systems are prepared by swelling in hot method. At first stage, poloxomers 407 and poloxomer 188 are added as portions into distilled water (70°C) in a beaker placed at a magnetic stirrer (homogenous-clear appearance). The prepared formulations are evaluated based on their gelling temperatures, whereby the temperature at which the system loses its flow properties is measured when the system is under magnetic stirring. At first stage, the formulations having been observed with gelling temperatures in the range of 31-45°C are selected. It is decided to observe the change in the gelling temperatures of these formulation after the addition of polymer which increases the adhesion.

### Step 2. Addition of Carbopol

Carbopol 934® is added to selected formulations at ratios 0.05%, 0.1%, 0.2% and 0.3% (w/w) 2%, 4% and 6% (w/w). The obtained data shows that the high concentrations of Carbopol 934® affect the gelling temperature significantly or creates problem in the preparation method. However, gelling temperature is found in the range of 35-31.6°C by the addition of 0.2%, 0.25% and 0.3%(w/w) Carbopol 934®. In the light of these data, it is decided to study the formulations comprising Carbopol 934® with an optimum amount of 0.2%, 0.25% and 0.3%(w/w) or below.

### Step 3. Addition ofActive Agents

Mixture of the active agents is added to the formulations whose gelling temperatures are selected at the end of step 2 and with the addition of Carbopol 934 (0.2%, 0.25% and %0.3 (w/w)) at the end of step 2. A decrease in the gelling temperatures of some formulations is observed during the addition of active agents. Final formulations are selected among the formulations having gelling temperatures around 34±2°C.

Moreover, it is decided to add a surface active agent to the formulations. Cremophor RH 40 and Tween 80 (TW) were selected and the concentrations of these surface active agents in the formulations were determined. Final formulations were prepared with decided polymer and surface active agent percentages. The parameters considered and results obtained in these studies are presented below.

There was no change in the gelling temperatures even after *in situ* gelling buccal formulations are kept at room temperature for 7 days.

*In situ* formulations comprising active agents at the optimized conditions based on the studies above are presented in the Table 1 below.

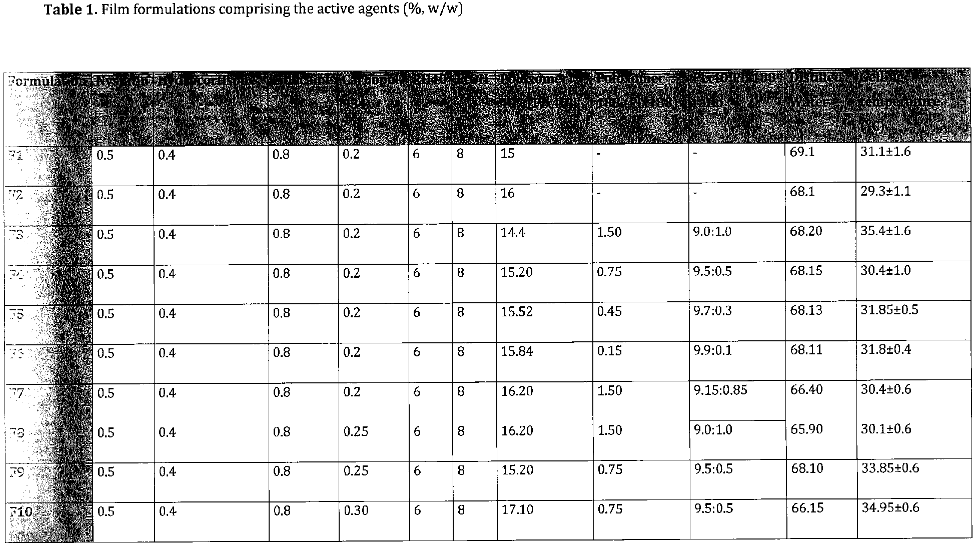

### Method of Manufacturing:

Poloxomer 188 and Poloxomer 407 polymers used in the relevant formulations are weighed at stated concentrations and added as portions into the distilled water (0-4°C) in a beaker placed in ice bath, mixed with the help of a magnetic stirrer for 2-4 hours by being kept in the ice bath. Following this, Poloxomer 407 was added to Poloxomer 188 and was mixed slightly without forming any air bubbles and let to be swelled in the refrigerator for 24 hours.

Then, lidocaine HCl is dissolved in a separate beaker in the remaining amount of water. Carbopol 934® was added to this mixture and mixed for at least 1 hour, and was allowed to swell during the night. Again, nystatin and hydrocortisone are allowed to dissolve in a separate beaker by taking a calculated amount of alcohol. The solubility of the active agents was increased by the addition of surface active agent to the same phase. The poloxomer mixture after waiting at 2-8°C overnight, the gel comprising Carbopol 934® and lidocaine is added and mixed homogenously by avoiding any air bubble formation. Then, a solution comprising active agents, alcohol and surface active agents are added to the gel mixture dropwise with the help of a dropper and final formulation is obtained.

### Preparation Method II (Hot Preparation)

Poloxomer 188, Poloxomer 407 and carbopol polymers used in the relevant formulations are weighed at stated concentrations and added as portions into the distilled water (70 °C) in a beaker placed. Then this polymer solutions mixed with the help of a magnetic stirrer for 30 minutes by being kept in beaker placed at 70°C then let to be swelled in the room temperature (24°C±0.5) for 24 hours.

Lidocaine HCl is dissolved in a separate beaker in the remaining amount of water. Again, nystatin and hydrocortisone are allowed to dissolve in a separate beaker by taking a calculated amount of alcohol. The solubility of the active agents was increased by the addition of surface active agent to the same phase. The poloxomer mixture after waiting at room temperature (24°C±0.5) overnight lidocaine is added and mixed homogenously by avoiding any air bubble formation. Then, a solution comprising active agents, alcohol and surface active agents are added to the gel mixture dropwise with the help of a dropper and final formulation is obtained.

## Claims

1. A pharmaceutical composition in buccal form comprising nystatin, at least one corticosteroid, at least one local anaesthetic, at least one *in situ* gelling polymer and at least one mucoadhesive polymer.

2. A pharmaceutical composition according to claim 1 wherein at least one corticosteroid is selected from the group consisting of hydrocortisone, cortisone, tixocortol, prednisolone, prednisone, methylprednisolone, triamcinolone, mometasone, amcinonide, budesonide, desonide, fluocinonide, fluosinilo, halcinonide, betamethasone, dexamethasone, fluocortolone.

3. A pharmaceutical composition according to claim 1 wherein at least one local anaesthetic is selected from the group consisting of mepivacaine, etidocaine, lidocaine, prilocaine, bupivacaine, and procaine.

4. A pharmaceutical composition according to claim 1 wherein the *in situ* gelling polymer is selected from the group consisting of gellan gum, alginic acid, xyloglucan, pectin, chitosan, poly (DL-lactic acid), poly(DL-lactide-co-glycolide), poly-caprolactone, poly(N-isopropyl acrylamide) [poly (NIPAAM)], polyoxyethylene, polyoxypropylene and combinations thereof.

5. A pharmaceutical composition according to claim 1 wherein the mucoadhesive polymer is selected from the group consisting of methyl cellulose, ethyl cellulose, hydroxyethyl cellulose, hydroxypropyl cellulose, hydroxypropyl methylcellulose, sodium carboxymethylcellulose, polyacrylic acid polymers including carbomers and polycarbophils, poly (hydroxyethyl methacrylate), poly (ethylene oxide), poly (vinyl pyrrolidone), poly (vinyl alcohol), sodium alginate, karaya gum, guar gum, xanthan gum, gelatine, pectin and chitosan and combinations thereof.

6. A pharmaceutical composition according to claim 2 wherein at least one corticosteroid is hydrocortisone acetate.

7. A pharmaceutical composition according to claim 3 wherein at least one local anaesthetic is lidocaine HCl salt and/or free base.

8. A pharmaceutical composition according to claim 4 wherein at least one *in situ* gelling polymer is a poloxomer mixture comprising Poloxomer 188 and Poloxomer 407.

9. A pharmaceutical composition according to claim 5 wherein at least one mucoadhesive polymer is a polyacrylic acid polymer.

10. A pharmaceutical composition according to claim 8 wherein the poloxomers forming *in situ* gelling polymer mixture have a poloxomer 407: poloxomer 188 ratio from 25:2 to 10:2 by weight.

11. A pharmaceutical composition according to claim 9 wherein the ratio of mucoadhesive polymer is in an amount from 0.1 to 5% by weight on the basis of the total weight of the composition.

12. A pharmaceutical composition according to claim 1 wherein the ratio of polymers used on the basis of the total weight of the composition is 10 to 25% by weight.

13. A pharmaceutical composition according to anyone of the preceding claims wherein said composition comprises at least one surface active agent in addition to *in situ* gelling polymers and mucoadhesive polymers.

14. A pharmaceutical composition according to claim 13 wherein at least one surface active agent is selected from a group comprising Tween 80, ascorbic palmitate and Cremophor RH 40 or combinations thereof.

15. A pharmaceutical composition according to claim 1 for use in the treatment of oral candidiasis or aphtha.

## Patentansprüche

1. Eine pharmazeutische Zusammensetzung in bukkaler Form, die Nystatin, zumindest ein Corticosteroid, zumindest ein Lokalanästhetikum, zumindest ein *in situ* gelierendes Polymer und zumindest ein mucoadhäsives Polymer aufweist.

2. Eine pharmazeutische Zusammensetzung gemäß Anspruch 1, wobei zumindest ein Corticosteroid aus der Gruppe ausgewählt ist, die aus Hydrocortison, Cortison, Tixocortol, Prednisolon, Prednison, Methylprednisolon, Triamcinolon, Mometason, Amcinonid, Budesonid, Desonid, Fluocinonid, Fluosinilo, Halcinonid, Betamethason, Dexamethason, Fluocortolon besteht.

3. Eine pharmazeutische Zusammensetzung gemäß Anspruch 1, wobei zumindest ein Lokalanästhetikum aus der Gruppe ausgewählt ist, die aus Mepivacain, Etidocain, Lidocain, Prilocain, Bupivacain und Procain besteht.

4. Eine pharmazeutische Zusammensetzung gemäß Anspruch 1, wobei das *in situ* gelierende Polymer aus der Gruppe ausgewählt ist, die aus Gellan-Gummi, Alginsäure, Xyloglucan, Pektin, Chitosan, Poly(DL-Milchsäure), Poly(DL-Lactid-coglycolid), Polycaprolacton, Poly(N-isopropylacrylamid) [Poly(NIPAAM)], Polyoxyethylen, Polyoxypropylen und Kombinationen daraus besteht.

5. Eine pharmazeutische Zusammensetzung gemäß Anspruch 1, wobei das mucoadhäsive Polymer aus der Gruppe ausgewählt ist, die aus Methylcellulose, Ethylcellulose, Hydroxyethylcellulose, Hydroxypropylcellulose, Hydroxypropylmethylcellulose, Natriumcarboxymethylcellulose, Polyacrylsäurepolymere einschließlich Carbomere und Polycarbophile, Poly(hydroxyethylmethacrylat), Poly(ethylenoxid), Poly(vinylpyrrolidon), Poly(vinylalkohol), Natriumalginat, Karaya-Gummi, Guar-Gummi, Xanthan-Gummi, Gelatine, Pektin und Chitosan und Kombinationen daraus besteht.

6. Eine pharmazeutische Zusammensetzung gemäß Anspruch 2, wobei zumindest ein Corticosteroid Hydrocortisonacetat ist.

7. Eine pharmazeutische Zusammensetzung gemäß Anspruch 3, wobei zumindest ein Lokalanästhetikum Lidocain, HCl-Salz und/oder freie Base, ist.

8. Eine pharmazeutische Zusammensetzung gemäß Anspruch 4, wobei zumindest ein *in situ* gelierendes Polymer ein Poloxamergemisch ist, das Poloxamer 188 und Poloxamer 407 aufweist.

9. Eine pharmazeutische Zusammensetzung gemäß Anspruch 5, wobei zumindest mucoadhäsives Polymer ein Polyacrylsäurepolymer ist.

10. Eine pharmazeutische Zusammensetzung gemäß Anspruch 8, wobei die Poloxamere, die ein *in situ* gelierendes Polymergemisch bilden, ein gewichtsmäßiges Verhältnis von Poloxamer 407 zu Poloxamer 188 in Höhe von 25:2 bis 10:2 aufweisen.

11. Eine pharmazeutische Zusammensetzung gemäß Anspruch 9, wobei das Verhältnis von mucoadhäsivem Polymer einen Betrag von 0,1 bis 5 Gew.-% auf Basis des Gesamtgewichts der Zusammensetzung ausmacht.

12. Eine pharmazeutische Zusammensetzung gemäß Anspruch 1, wobei das Verhältnis der verwendeten Polymere auf Basis des Gesamtgewichts der Zusammensetzung 10 bis 25 Gew.-% beträgt.

13. Eine pharmazeutische Zusammensetzung gemäß einem der vorhergehenden Ansprüche, wobei die Zusammensetzung zusätzlich zu *in situ* gelierenden Polymeren und mucoadhäsiven Polymeren zumindest ein oberflächenaktives Mittel aufweist.

14. Eine pharmazeutische Zusammensetzung gemäß Anspruch 13, wobei zumindest ein oberflächenaktives Mittel aus einer Gruppe ausgewählt ist, die aus Tween 80, Ascorbinpalmitat und Cremophor RH 40 oder Kombinationen daraus besteht.

15. Eine pharmazeutische Zusammensetzung gemäß Anspruch 1 zur Verwendung bei der Behandlung von oraler Candidiasis oder Aphthen.

## Revendications

1. Composition pharmaceutique dans une forme pour l'administration par voie buccale, comprenant de la nystatine, au moins un corticostéroïde, au moins un anesthésique local, au moins un polymère gélifiant *in situ* et au moins un polymère muco-adhésif.

2. Composition pharmaceutique selon la revendication 1, dans laquelle au moins un corticostéroïde est choisi dans le groupe consistant en hydrocortisone, cortisone, tixocortol, prednisolone, prednisone, méthylprednisolone, triamcinolone, mométasone, amcinonide, budésonide, désonide, fluocinonide, fluosinilo, halcinonide, bétaméthasone, dexaméthasone, fluocortolone.

3. Composition pharmaceutique selon la revendication 1, dans laquelle au moins un anesthésique local est choisi dans le groupe consistant en mépivacaïne, étidocaïne, lidocaïne, prilocaïne, bupivacaïne et procaïne.

4. Composition pharmaceutique selon la revendication 1, dans laquelle le polymère gélifiant *in situ* est choisi dans le groupe consistant en gomme gellane, acide alginique, xyloglucane, pectine, chitosane, poly(acide DL-lactique), poly(DL-lactide-co-glycolide), polycaprolactone, poly(N-isopropyl acrylamide)[poly(NIPAAM)], polyoxyéthylène, polyoxypropylène et leurs combinaisons.

5. Composition pharmaceutique selon la revendication 1, dans laquelle le polymère muco-adhésif est choisi dans le groupe consistant en méthyl cellulose, éthyl cellulose, hydroxyéthyl cellulose, hydroxypropyl cellulose, hydroxypropyl méthylcellulose, carboxyméthylcellulose sodique, polymères d'acide polyacrylique comprenant les carbomères et les polycarbophiles, poly(méthacrylate d'hydroxyéthyle), poly(oxyde d'éthylène), poly(vinyl pyrrolidone), poly(alcool vinylique), alginate de sodium, gomme karaya, gomme de guar, gomme xanthane, gélatine, pectine et chitosane et leurs combinaisons.

6. Composition pharmaceutique selon la revendication 2, dans laquelle au moins un corticostéroïde est l'acétate d'hydrocortisone.

7. Composition pharmaceutique selon la revendication 3, dans laquelle au moins un anesthésique local est la lidocaïne HCl sel et/ou base libre.

8. Composition pharmaceutique selon la revendication 4, dans laquelle au moins un polymère gélifiant *in situ* est un mélange de poloxamères comprenant le Poloxamère 188 et le Poloxamère 407.

9. Composition pharmaceutique selon la revendication 5, dans laquelle au moins un polymère muco-adhésif est un polymère d'acide polyacrylique.

10. Composition pharmaceutique selon la revendication 8, dans laquelle les poloxamères formant un mélange de polymères gélifiant *in situ* ont un rapport poloxamère 407:poloxamère 188 de 25:2 à 10:2 en poids.

11. Composition pharmaceutique selon la revendication 9, dans laquelle le rapport de polymère muco-adhésif est dans une quantité de 0,1 à 5 % en poids sur la base du poids total de la composition.

12. Composition pharmaceutique selon la revendication 1, dans laquelle le rapport de polymères utilisés sur la base du poids total de la composition est de 10 à 25 % en poids.

13. Composition pharmaceutique selon l'une quelconque des revendications précédentes, dans laquelle ladite composition comprend au moins un agent tensio-actif en plus des polymères gélifiant *in situ* et des polymères muco-adhésifs.

14. Composition pharmaceutique selon la revendication 13, dans laquelle au moins un agent tensio-actif est choisi dans un groupe comprenant le Tween 80, le palmitate ascorbique et le Crémophore RH 40 ou leurs combinaisons.

15. Composition pharmaceutique selon la revendication 1 pour une utilisation dans le traitement de la candidose orale ou de l'aphte.
